# EUROPÄISCHE PATENTSCHRIFT

(11) **EP 0 297 446 B1**
(45) Veröffentlichungstag und Bekanntmachung des Hinweises auf die Patenterteilung: **02.06.1993**
(21) Anmeldenummer: 88110066.3
(22) Anmeldetag: 24.06.1988
(51) Int. Cl.: C07D 207/06, C07D 211/02, C07D 211/10

(54) **Verfahren zur Herstellung von N-Heterocyclen**
Process for the preparation of N-heterocycles
Procédé de préparation d'hétérocycles azotés

(30) Priorität: 01.07.1987 DE 3721777
(43) Veröffentlichungstag der Anmeldung: 04.01.1989
(73) Patentinhaber: BASF Aktiengesellschaft, 67063 Ludwigshafen (DE)
(72) Erfinder: Hesse, Michael, Dr., D-6700 Ludwigshafen (DE); Hoelderich, Wolfgang, Dr., D-6710 Frankenthal (DE); Schwarzmann, Matthias, Dr., D-6703 Limburgerhof (DE)

(56) Entgegenhaltungen:
- DE-A- 3 326 579
- DE-A- 3 327 000
- US-A- 4 375 002

## Beschreibung

Die vorliegende Erfindung betrifft ein Verfahren zur Herstellung von N-Heterocyclen durch Umsetzung von Olefinen der Formeln (III) und (IV) mit NH₃ oder primären Aminen in Gegenwart von Zeolithen als Katalysatoren.

Gesättigte N-Heterocyclen wie Pyrrolidine oder Piperidine sind in der Wirkstoffchemie gesuchte Ausgangsstoffe, die sich auf mehreren Wegen herstellen lassen (Bird,Cheeseman: Comprehensive Heterocyclic Chemistry 4, S. 89-154 und Boulton,Mc Killop: Comprehensive Heterocyclic Chemistry 2, S. 67-98). Schwierig gestaltet sich jedoch die Herstellung von Verbindungen der obengenannten Stoffklasse, bei denen das Stickstoffatom durch α-ständige sperrige Gruppen abgeschirmt wird, wie z.B. 2,2,6,6-Tetramethylpiperidin. Diese Stoffe sind in einigen speziellen Fällen zugänglich durch Umsetzung von Kondensationsprodukten des Acetons, wie z.B. Phoron, mit Ammoniak (DE 2 412 750, DE 2 352 127). Hierbei wird NH₃ an α,β-ungesättigte Carbonylverbindungen unter Ringschluß addiert. Die Carbonylfunktion muß dann separat hydriert werden, um zu den gewünschten Produkten zu gelangen. Dies bedingt eine mehrstufige Prozeßführung. Hingegen ist die Umsetzung von nicht aktivierten Olefinen auf diesem Wege nicht möglich.

Es ist bekannt, daß sich Amine mit Olefinen, vorzugsweise mit an der Doppelbindung substituierten Olefinen, an sauren Ionenaustauschern (US 4 536 602) und an Zeolithen zu substituierten Aminen umsetzen lassen (DE 3 326 579, DE 3 327 000 und US 4 375 002, EP 39 918, EP 77 016, EP 101 921).

Hierbei wird jedoch nur die NH₃-Addition an Monoolefine oder nur die NH₃-Addition an eine Doppelbindung einer mehrfach ungesättigten Verbindung beschrieben. Die Bildung cyclischer Amine durch intramolekulare Reaktion zweier Doppelbindungen wird hierbei nicht offenbart.

Es ergab sich die Aufgabe, ein Verfahren zu finden, nach dem sterisch anspruchsvoll substituierte 5- und 6-gliedrige N-Heterocyclen, wie in Formel (I) und (II) beschrieben, selektiv herzustellen sind, ohne die Notwendigkeit mehrstufiger Reaktionsführung.

Es wurde nun überraschenderweise gefunden, daß bei der Umsetzung von nichtkonjugierten Diolefinen mit Ammoniak oder primären Aminen in Gegenwart von Zeolithen als Katalysatoren gesättigte N-Heterocyclen anfallen, entstanden durch Addition des Amins an beide Doppelbindungen unter Cyclisierung.

Gegenstand der Erfindung ist ein Verfahren zur Herstellung von N-Heterocyclen der allgemeinen Formeln (I) und (II) 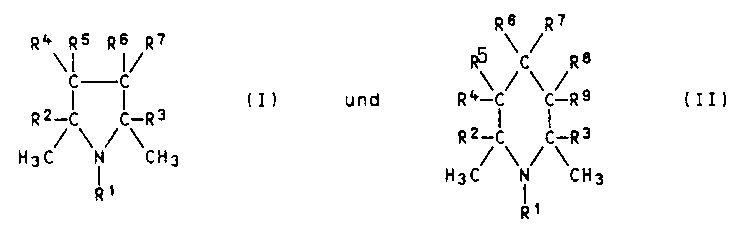
in denen R¹ und R⁴ bis R⁹ Wasserstoff oder eine Alkyl-, Aryl-, Aralkyl-, Alkaryl- oder Cycloalkylgruppe bedeuten kann und R² und R³ eine Alkyl-, Alkaryl- oder Cycloalkylgruppe bedeuten kann, bei dem man Olefine der Formeln (III) und (IV) 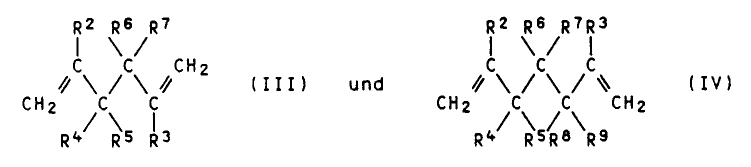
in denen R² bis R⁹ obige Bedeutungen annehmen können, mit NH₃ oder einem primären Amin der Formel H₂NR¹, wobei R¹ obige Bedeutung hat in Gegenwart von Zeolithen als Katalysatoren bei Temperaturen von 50 bis 500°C umgesetzt werden.

Olefine der Formeln (III) und (IV), die erfindungsgemäß als Ausgangsstoffe bevorzugt umgesetzt werden können sind z.B. 2,5-Dimethyl-1,5-hexadien und 2,6-Dimethyl-1,6-dimethylheptadien.

Beispiele für die erfindungsgemäß umgesetzten Amine sind z.B. Ammoniak, Methylamin, Ethylamin, Cyclopentylamin, Cyclohexylamin, Anilin und Toluidine.

Als Katalysatoren für das erfindungsgemäße Verfahren verwendet man Zeolithe, insbesondere vom Pentasiltyp. Die Zeolithe werden zweckmäßig in der aciden Form angewendet. Zeolithe sind kristalline Aluminiumsilikate, die eine hochgeordnete Struktur mit einem starren dreidimensionalen Netzwerk von SiO4- und AlO₄-Tetraedern besitzen, die durch gemeinsame Sauerstoffatome verbunden sind. Das Verhältnis der Si- und Al-Atome zu Sauerstoff beträgt 1:2. Die Elektrovalenz der Aluminium enthaltenden Tetraeder ist durch Einschluß von Kationen in den Kristall, z.B. eines Alkali- oder Wasserstoffions ausgeglichen, Ein Kationenaustausch ist möglich. Die Räume zwischen den Tetraedern sind vor der Dehydration durch Trocknen bzw. Calcinieren von Wassermolekülen besetzt.

In den Zeolithen können anstelle von Aluminium auch andere Elemente wie B, Ga, Fe, Cr, V, As, Sb, Bi oder Be oder deren Gemische in das Gitter eingebaut werden, oder das Silicium kann durch ein vierwertiges Element wie Ge, Ti, Zr, Hf ersetzt werden.

Entsprechend ihrer Struktur werden Zeolithe in verschiedene Gruppen unterteilt. So bilden bei der Mordenit-Gruppe Ketten oder bei der Chabasit-Gruppe Schichten aus Tetraedern die Zeolith-Struktur, während sich bei der Faujasit-Gruppe die Tetraeder zu Polyedern ordnen, z.B. in Form eines Kubooktaeders, der aus Vierringen bzw. Sechsringen aufgebaut ist. Je nach Verknüpfung der Kubooktaeder, wodurch unterschiedlich große Hohlräume und Poren entstehen, unterscheidet man in Zeolithe vom Typ A, L, X oder Y.

Für das erfindungsgemäße Verfahren in Betracht kommende Katalysatoren sind z.B. Zeolithe aus der Mordenit-Gruppe oder engporige Zeolithe vom Erionit-, Offretit- bzw. Chabasit-Typ oder Zeolithe vom Faujasit-Typ, z.B. Y-, X- oder L-Zeolithe.

In diese Gruppen von Zeolithen gehören auch die sogenannten "ultra-stabilen" Zeolithe des Faujasit-Typs, d.h. dealuminierte Zeolithe. Verfahren zur Herstellung solcher Zeolithe sind mannigfach beschrieben.

Besonders vorteilhaft sind Zeolithe vom Pentasiltyp. Diese haben als Grundbaustein einen aus SiO₄-Tetraedern aufgebauten Fünfring gemeinsam. Sie sind durch ein hohes SiO₂/Al₂O₃-Verhältnis gekennzeichnet sowie durch Porengrößen, die zwischen denen der Zeolithe vom Typ A und denen vom Typ X oder Y liegen.

Diese Zeolithe können unterschiedliche chemische Zusammensetzungen aufweisen. Es handelt sich hierbei um Alumino-, Boro-, Eisen-, Beryllium-, Gallium-, Chrom-, Arsen-, Antimon- und Wismutsilikatzeolithe oder deren Gemische sowie Alumino-, Boro-, Gallium- und Eisengermanatzeolithe oder deren Gemische. Insbesondere eignen sich die Alumino-, Boro- und Eisensilikatzeolithe des Pentasiltyps für das erfindungsgemäße Verfahren. Der Aluminosilikatzeolith wird z.B. aus einer Aluminiumverbindung, vorzugsweise Al(OH)₃ oder Al₂(SO₄)₃ und einer Siliciumkomponente, vorzugsweise hochdispersem Siliciumdioxid in wäßriger Aminlösung, insbesondere in Polyaminen wie 1,6-Hexandiamin- oder 1,3-Propandiamin- oder Triethylentetramin-Lösung mit oder insbesondere ohne Alkali- oder Erdalkalizusatz bei 100 bis 220°C unter autogenem Druck hergestellt. Hierzu gehören auch die isotaktischen Zeolithe nach EP 34 727 und EP 46 504. Die erhaltenen Aluminosilikatzeolithe enthalten je nach Wahl der Einsatzstoffmengen ein SiO₂/Al₂O₃-Verhältnis von 10 bis 40.000. Aluminosilikatzeolithe des Pentasiltyps können auch in etherischem Medium wie Diethylenglykoldimethylether, in alkoholischem Medium wie Methanol bzw. 1,4-Butandiol oder in Wasser synthetisiert werden.

Zu den erfindungsgemäß verwendbaren siliciumreichen Zeolithen (SiO₂/Al₂O₃ ≧ 10) gehören auch die verschiedenen ZSM-Typen, Ferrierit, Nu-1 und Silicalit®.

Borosilikatzeolithe kann man z.B. bei 90 bis 200°C unter autogenem Druck synthetisieren, indem man eine Borverbindung, z.B. H₃BO₃, mit einer Siliciumverbindung, vorzugsweise hochdispersem Siliciumdioxid in wäßriger Aminlösung, insbesondere in 1,6-Hexandiamin- oder 1,3-Propandiamin- oder Triethylentetramin-Lösung mit und insbesondere ohne Alkali- oder Erdalkalizusatz zur Reaktion bringt. Hierzu gehören auch die isotaktischen Zeolithe nach EP 34 727 und EP 46 504. Solche Borosilikatzeolithe können ebenfalls hergestellt werden, wenn man die Reaktion statt in wäßriger Aminlösung in etherischer Lösung, z.B. Diethylenglykoldimethylether oder in alkoholischer Lösung, z.B. 1,6-Hexandiol durchführt.

Den Eisensilikatzeolith erhält man z.B. aus einer Eisenverbindung, vorzugsweise Fe₂(SO₄)₃ und einer Siliciumverbindung, vorzugsweise hochdispersem Siliciumdioxid in wäßriger Aminlösung, insbesondere 1,6-Hexandiamin, mit oder ohne Alkali- oder Erdalkalizusatz bei 100 bis 200°C unter autogenem Druck.

Die so hergestellten Alumino-, Boro und Eisensilikatzeolithe können nach ihrer Isolierung, Trocknung bei 100 bis 160°C, vorzugsweise 110°C und Calcinierung bei 450 bis 550°C, vorzugsweise 500°C, mit einem BindemittelL im Verhältnis 90:10 bis 40:60 Gew.% zu Strängen oder Tabletten verformt werden. Als Bindemittel eignen sich diverse Aluminiumoxide, bevorzugt Boehmit, amorphe Aluminosilikate mit einem SiO₂/Al₂O₃-Verhältnis von 25:75 bis 90:5, bevorzugt 75:25, Siliciumdioxid, bevorzugt hochdisperses SiO₂, Gemische auch hochdispersem SiO₂ und hochdispersem Al₂O₃, TiO₂, ZrO₂ sowie Ton. Nach der Verformung werden die Extrudate oder Preßlinge bei 110°C/16 h getrocknet und bei 500°C/16 h calciniert.

Man erhält auch sehr wirksame Katalysatoren, wenn der isolierte Alumino-bzw. Borosilikatzeolith direkt nach der Trocknung verformt wird und erst nach der Verformung einer Calcinierung unterworfen wird. Die hergestellten Alumino- und Borosilikatzeolithe können in reiner Form, ohne Binder, als Stränge oder Tabletten eingesetzt werden, wobei als Verstrangungs- oder Peptisierungshilfsmittel, z.B. Ethylcellulose, Stearinsäure, Kartoffelstärke, Ameisensäure, Oxalsäure, Essigsäure, Salpetersäure, Ammoniak, Amine, Silikoester und Graphit oder deren Gemische verwendet werden. Liegt der Zeolith aufgrund der Art seiner Herstellung nicht in der katalytisch aktiven, aciden H-Form vor, sondern z.B. in der Na-Form, dann kann diese durch Ionenaustausch, z.B. mit Ammoniumionen und anschließende Calcinierung oder durch Behandlung mit Säuren vollkommen oder partiell in die gewünschte H-Form überführt werden.

Um eine möglichst hohe Selektivität, hohe Umsätze sowie lange Standzeiten zu erreichen, ist es vorteilhaft, die Zeolithe zu modifizieren. Eine geeignete Modifizierung der Katalysatoren besteht z.B. darin, daß man den unverformten oder verformten Zeolithen mit Metallsalzen durch einen Ionenaustausch oder durch Imprägnierung dotiert. Als Metalle werden Alkalimetalle wie Li, Cs, K, Erdalkalimetall wie Mg, Ca, Sr, Ba, Metalle der 3., 4. und 5. Hauptgruppe wie B, Al, Ga, Ge, Sn, Pb, Bi, Seltene Erdmetalle wie La, Ce, Pr, Nd, Er, Yb und U oder Edelmetalle eingesetzt. Die Modifizierung mit diesen Metallen kann durch Ionenaustausch oder Imprägnierung erfolgen.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder Wasserdampf unterwirft.

Zweckmäßigerweise führt man diese Dotierung so durch, daß man den verformten Zeolithen in einem Steigrohr vorlegt und bei 20 bis 100°C eine wäßrige oder ammoniakalische Lösung eines Halogenids oder eines Nitrats der voranbeschriebenen Metalle überleitet. Ein derartiger Ionenaustausch kann an der Wasserstoff-, Ammonium- und Alkaliform des Zeolithen vorgenommen werden. Eine weitere Möglichkeit der Metallaufbringung auf den Zeolithen ist gegeben, indem man das zeolithische Material, z.B. mit einem Halogenid, einem Nitrat oder einem Oxid der voranbeschriebenen Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform besteht z.B. darin, daß man Cu(NO₃)₂ x 3 H₂O oder Ni(NO₃)₂ x 6 H₂O oder Ce(NO₃)₃ x 6 H₂O oder Cr(NO₃)₃ x 6 H₂O oder Pd(NO₃)₂ in Wasser löst und mit dieser Lösung den verformten oder unverformten Zeolith eine gewisse Zeit, z.B: 30 Minuten, tränkt. Die eventuell überstehende Lösung wird im Rotationsverdampfer von Wasser befreit. Danach wird der getränkte Zeolith bei etwa 150°C getrocknet und bei etwa 550°C calciniert. Dieser Tränkvorgang kann mehrmals hintereinander vorgenommen werden, um den gewünschten Metallgehalt einzustellen.

Es ist auch möglich, eine wäßrige Ni(NO₃)₂-Lösung oder ammoniakalische Pd(NO₃)₂-Lösung herzustellen und darin den reinen pulverförmigen Zeolithen bei 40 bis 100°C unter Rühren etwa 24 h aufzuschlämmen. Nach Abfiltrieren, Trocknen bei etwa 150°C und Calcinierung bei etwa 500°C kann das so gewonnene zeolithische Material mit oder ohne Bindemittel zu Strängen, Pellets oder Wirbelgut weiterverarbeitet werden.

Ein Ionenaustausch des in der H-Form oder Ammonium-Form oder Alkali-Form vorliegenden Zeolithen kann so vorgenommen werden, daß man den Zeolithen in Strängen oder Pellets in einer Kolonne vorlegt und darüber z.B. eine wäßrige Ni(NO₃)₂-Lösung oder ammoniakalische Pd(NO₃)₂-Lösung bei leicht erhöhter Temperatur zwischen 30 und 80°C im Kreislauf 15 bis 20 h leitet. Danach wird mit Wasser ausgewaschen, bei etwa 150°C getrocknet und bei etwa 550°C calciniert.

Bei manchen metalldotierten Zeolithen, z.B. Pd-, Cu-, Ni-dotierten Zeolithen ist eine Nachbehandlung mit Wasserstoff vorteilhaft.

Im einzelnen geht man vorteilhaft so vor, daß man die Zeolithe in Pulverform mit 1 n Phosphorsäure 1 Stunde bei 80°C behandelt. Nach der Behandlung wird mit Wasser gewaschen, bei 110°C/16 Stunden getrocknet und bei 500°C/20 Stunden calciniert. Nach einer anderen Arbeitsweise behandelt man die Zeolithe vor oder nach ihrer Verformung mit Bindemittel, z.B. 1 bis 3 Stunden bei Temperaturen von 60 bis 80°C mit einer 3 bis 25 gew.%igen, insbesondere 12 bis 20 gew.%igen wäßrigen Salzsäure. Anschließend wird der so behandelte Zeolith mit Wasser gewaschen, getrocknet und bei 400 bis 500°C calciniert.

Eine besondere Ausführungsform für die Säurebehandlung besteht darin, daß man das zeolithische Material vor seiner Vorformung bei erhöhter Temperatur mit Flußsäure, die im allgemeinen als 0,001 n bis 2 n, vorzugsweise 0,05 n bis 0,5 n Flußsäure eingesetzt wird, behandelt, beispielsweise durch Erhitzen unter Rückfluß über einen Zeitraum von 0,5 bis 5, vorzugsweise 1 bis 3 h. Nach Isolierung, z.B. durch Abfiltrieren und Auswaschen des zeolithischen Materials, wird dieses zweckmäßig bei Temperaturen von 100 bis 160°C getrocknet und bei Temperaturen von 450 bis 600°C calciniert. Gemäß einer weiteren bevorzugten Ausführungsform für die Säurebehandlung wird das zeolithische Material nach einer Verformung mit Bindemittel bei erhöhter Temperatur, zweckmäßig bei Temperaturen von 50 bis 90°C, vorzugsweise 60 bis 80°C, über einen Zeitraum von 0,5 bis 5 h, vorzugsweise mit 12 bis 20 gew.%iger Salzsäure, behandelt. Zweckmäßig wird das zeolithische Material anschließend ausgewaschen, bei Temperaturen von 100 bis 160°C getrocknet und bei Temperaturen von 450 bis 600°C calciniert. Einer HF-Behandlung kann sich auch eine HCl-Behandlung anschließen.

Nach einer anderen Arbeitsweise kann man Zeolithe durch Aufbringen von Phosphorverbindungen, wie Trimethoxiphosphat, Trimethoxiphosphin, primärem, sekundärem oder tertiärem Natriumphosphat modifizieren. Besonders vorteilhaft hat sich die Behandlung mit primären Natriumphosphat erwiesen. Hierbei werden die Zeolithe in Strang-, Tabletten- oder Wirbelgut-Form mit wäßriger NaH₂PO₄-Lösung getränkt, bei 110°C getrocknet und bei 500°C calciniert.

Wenn bei der erfindungsgemäßen Verwendung der zeolithischen Katalysatoren eine durch Koksabscheidung bedingte Desaktivierung eintritt, empfiehlt es sich, diese durch Abbrennen der Koksablagerung mit Luft oder mit einem Luft/N₂-Gemisch bei 400 bis 550°C, bevorzugt 500°C, zu regenerieren. Die Zeolithe erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen.

Die hier beschriebenen Katalysatoren können wahlweise als 2- bis 4 mm-Stränge oder als Tabletten mit 3 bis 5 mm Durchmesser oder als Splitt mit Teilchengrößen von 0,1 bis 0,5 mm oder als Wirbelkontakt eingesetzt werden.

Die für die erfindungsgemäße Umsetzung in der Regel gewählten Reaktionsbedingungen sind 50 bis 500°C, z.B. 150 bis 450°C und insbesondere 200 bis 400°C und eine Katalysatorbelastung WHSV von 0,1 bis 20 h⁻¹, insbesondere von 1,0 bis 10,0 h⁻¹ g Ausgangsstoff je g Katalysator und Stunde.

Die Reaktion kann sowohl in einem Festbett als auch in einem Wirbelbett ausgeführt werden.

Die Umsetzung wird im allgemeinen bei einem Druck zwischen 50 und 500 bar, insbesondere zwischen 150 und 350 bar, durchgeführt.

Das Molverhältnis der Einsatzstoffe Amin/Olefin liegt in der Regel zwischen 10:1 und 1:10, vorzugsweise zwischen 3:1 und 1:2.

Das Verfahren wird vorzugsweise kontinuierlich durchgeführt. Man kann aber auch diskontinuierlich arbeiten.

Schwerflüchtige oder feste Ausgangsstoffe kann man in gelöster Form, z.B. in THF-, Toluol- oder Petrolether-Lösung einsetzen. Es ist auch eine Verdünnung des Ausgangsstoffes mit derartigen Lösungsmitteln möglich.

Nach der Umsetzung werden die entstandenen Produkte durch übliche Verfahren, z.B. durch Destillation oder Extraktion, aus dem Reaktionsgemisch isoliert und nötigenfalls mittels weiterer Trennoperationen auf die gewünschte Reinheit gebracht; nichtumgesetzte Ausgangsstoffe können in den Reaktor zurückgeführt werden. Auch die bei der Reaktion anfallenden Nebenprodukte wie z.B. die nichtcyclischen Monoadditionsprodukte der eingesetzten Amine können vorteilhaft entweder einer anderweitigen technischen Nutzung oder einer nachfolgenden vollständigen Cyclisierung zugeführt werden.

Für die Beispiele werden folgende Katalysatoren eingesetzt.

### Katalysator A

Ein Borosilikatzeolith des Pentasil-Typs wird in einer hydrothermalen Synthese aus 640 g hochdispersem SiO₂, 122 g H₃BO₃, 8000 g einer wäßrigen 1,6-Hexandiam-Lösung (Mischung 50:50 Gew.%) bei 170°C unter autogenem Druck in einem Rührautoklaven hergestellt. Nach Abfiltrieren und Auswaschen wird das kristalline Reaktionsprodukt bei 100°C/24 h getrocknet und bei 500°C/24 h calciniert. Dieser Borosilikatzeolith setzt sich zusammen aus 94,2 Gew.% SiO₂ und 2,3 Gew.% B₂O₃. Daraus werden durch Verformen mit Boehmit im Gewichtsverhältnis 60:40 2 mm-Stränge hergestellt, die bei 110°C/16 h getrocknet und bei 500°C/24 h calciniert werden.

### Katalysator B

Katalysator B erhält man, indem man Katalysator A mit einer wäßrigen Cr(NO₃)₃-Lösung imprägniert, danach bei 130°C/2 h trocknet und bei 500°C/24 h calciniert. Der Cr-Gehalt beträgt 2,5 Gew.%.

### Katalysator C

Katalysator C wird wie Katalysator B hergestellt, jedoch mit einer wäßrigen Lösung aus Ce-Nitrat statt Cr-Nitrat getränkt. Der Ce-Gehalt beträgt 7,1 Gew.%.

### Beispiele 1 bis 3

In einem 0,3 l-Rührautoklaven werden jeweils 10 ml eines der oben beschriebenen Katalysatoren gegeben und die Edukte eingefüllt. Soweit die Edukte gasförmig sind, werden sich nach Verschließen des Autoklaven aufgedrückt. Die Menge an Einsatzprodukt wird so bemessen, daß bei der gewählten Reaktionstemperatur der gewünschte Druck als Eigendruck erreicht wird. Ist das nicht möglich, wird mit Stickstoff der gewünschte Druck eingestellt.

### Beispiele 4 und 5

Die Reaktion wird unter isothermen Bedingungen in einem Rohrreaktor (Wendel, 0,6 cm Innendurchmesser, 90 cm Länge) mindestens 6 Stunden lang durchgeführt. Die Reaktionsprodukte werden durch übliche Methoden abgetrennt und charakterisiert. Die quantitative Bestimmung der Reaktionsprodukte und der Ausgangsstoffe erfolgt gaschromatographisch.

In Tabelle I sind die Versuchsergebnisse der Beispiele 1 bis 5 zusammengefaßt.

**Tabelle I**

| Bsp. | Katalysator | Druck [bar] | Temperatur [°C] | Verhältnis | | Selektivität | |
|---|---|---|---|---|---|---|---|
| | | | | Dien/NH₃ | Umsatz [%] | TMP* [%] | offenkettige Vorstufe [%] |
| 1 | A | 300 | 300 | 1:3 | 32 | 16,7 | 32,5 |
| 2 | B | 300 | 300 | 1:3 | 36 | 15,5 | 27,0 |
| 3 | C | 300 | 300 | 1:3 | 24 | 24,9 | 31,0 |
| 4 | A | 300 | 300 | 1:2,5 | 30,1 | 16,0 | 23,2 |
| 5 | B | 300 | 300 | 1:2,5 | 38,9 | 9,9 | 19,7 |

| | | | | | | | |
|---|---|---|---|---|---|---|---|
| *TMP = 2,2,5,5-Tetramethylpyrrolidin | | | | | | | |

Die einzigen selektivitätsmindernden Nebenprodukte der Umsetzung sind isomerisierte Octadiene. Dimerisationsprodukte oder andere Amine werden nicht gefunden.

## Patentansprüche

1. Verfahren zur Herstellung von N-Heterocyclen der allgemeinen Formeln (I) und (II) 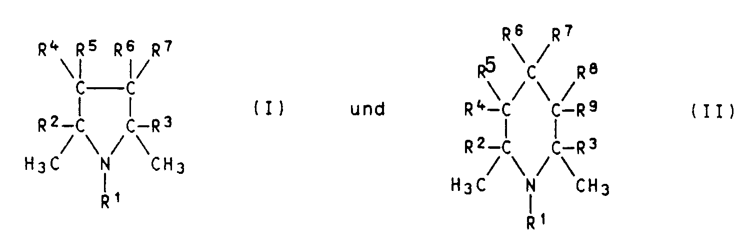 in denen R¹ und R⁴ bis R⁹ Wasserstoff oder eine Alkyl-, Aryl-, Aralkyl-, Alkaryl- oder Cycloalkylgruppe bedeuten kann und R² und R³ eine Alkyl-, Alkaryl- oder Cycloalkylgruppe bedeuten kann, dadurch gekennzeichnet, daß man Olefine der Formeln (III) und (IV) 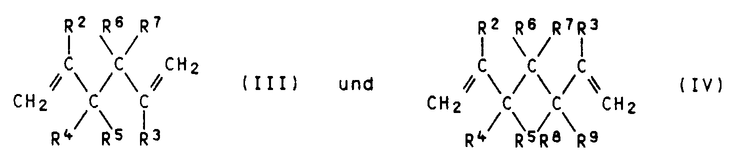 in denen R² bis R⁹ obige Bedeutungen annehmen können, mit NH₃ oder einem primären Amin der Formel H₂NR¹, wobei R¹ obige Bedeutung hat, in Gegenwart von Zeolithen als Katalysatoren bei Temperaturen von 50 bis 500°C umgesetz werden.

2. Verfahren nach Anspruch 1, dadurch gekennzeichnet, daß als Olefine 2,5-Dimethyl-1,5-hexadien oder 2,6-Dimethyl-2,6-heptadien eingesetzt werden.

3. Verfahren nach Ansprüchen 1 und 2, dadurch gekennzeichnet, daß man als Katalysatoren Zeolithe des Pentasiltyps verwendet.

4. Verfahren nach Anspruch 3, dadurch gekennzeichnet, daß man als Katalysatoren Aluminosilikatzeolithe des Pentasiltyps verwendet.

5. Verfahren nach Anspruch 3, dadurch gekennzeichnet, daß man als Katalysatoren Borosilikatzeolithe des Pentasiltyps verwendet.

6. Verfahren nach Anspruch 3, dadurch gekennzeichnet, daß man als Katalysatoren Eisensilikatzeolithe des Pentasiltyps verwendet.

7. Verfahren nach Anspruch 3, dadurch gekennzeichnet, daß man als Katalysatoren Aluminiumsilikatzeolithe des Faujasit-, Erionit-, Chabasit- und Offretit-Typs verwendet.

8. Verfahren nach den Ansprüchen 1 bis 7, dadurch gekennzeichnet, daß man Zeolithkatalysatoren verwendet, die mit Übergangsmetallen und/oder Edelmetallen und/oder Seltenen Erden, und/oder Alkalimettalen und/oder Erdalkalimetallen dotiert sind.

## Claims

1. A process for the preparation of an N-heterocycle of the formula (I) or (II) 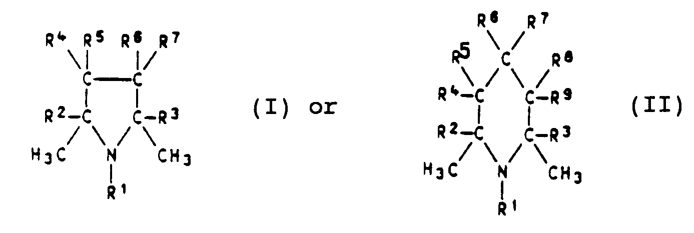 where R¹ and R⁴ to R⁹ are each hydrogen, alkyl, aryl, aralkyl, alkylaryl or cycloalkyl and R² and R³ are each an alkyl, alkylaryl or cycloalkyl group, wherein an olefin of the formula (III) or (IV) 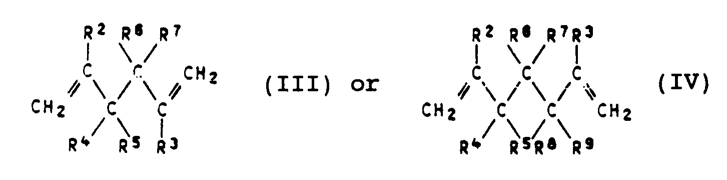 where R² to R⁹ have the above meanings, is reacted at from 50 to 500°C with NH₃ or with a primary amine of the formula H₂NR¹, where R¹ has the above meanings, in the presence of a zeolite as a catalyst.

2. A process as claimed in claim 1, wherein the olefin used is 2,5-dimethyl-1,5-hexadiene or 2,6-dimethyl-2,6-heptadiene.

3. A process as claimed in claim 1 or 2, wherein the catalyst used is a zeolite of the pentasil type.

4. A process as claimed in claim 3, wherein the catalyst used is an aluminosilicate zeolite of the pentasil type.

5. A process as claimed in claim 3, wherein the catalyst used is a borosilicate zeolite of the pentasil type.

6. A process as claimed in claim 3, wherein the catalyst used is an iron silicate zeolite of the pentasil type.

7. A process as claimed in claim 3, wherein the catalyst used is an aluminosilicate zeolite of the faujasite, erionite, chabasite or offretite type.

8. A process as claimed in any of claims 1 to 7, where in the zeolite catalyst used has been doped with transition metals and/or noble metals and/or rare earth metals and/or alkali metals and/or alkaline earth metals.

## Revendications

1. Procédé de préparation d'hétérocycles azotés de formules générales (I) et (II) 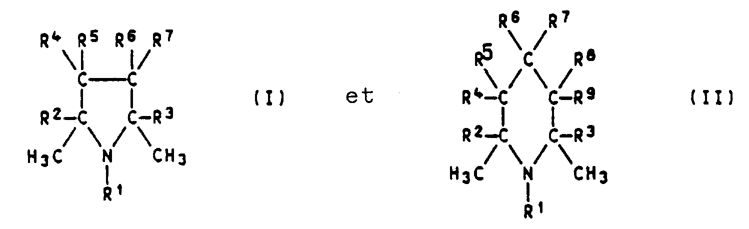 dans lesquelles R¹ et R⁴ à R⁹ peuvent représenter chacun un atome d'hydrogène au un groupement alkyle, aryle, aralkyle, alkylaryle ou cycloalkyle et R² et R³ peuvent représenter chacun un groupement alkyle, alkylaryle ou cycloalkyle, caractérisé en ce qu'on fait réagir des oléfines de formules (III) et (IV) 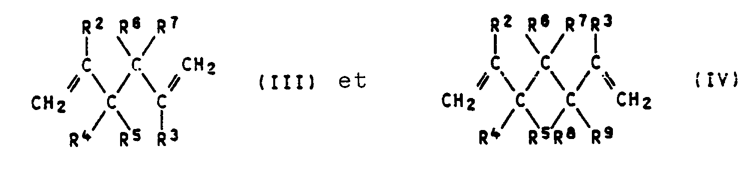 dans lesquelles R² à R⁹ peuvent prendre les significations sus-indiquées, avec NH₃ ou une amine primaire de formule H₂NR¹, R¹ ayant la signification sus-indiquée, en présence de zéolithes servant de catalyseur et à des températures de 50 à 500°C.

2. Procédé selon la revendication 1, caractérisé en ce qu'on met en réaction, en tant qu'oléfine, du 2,5-diméthyl-1,5-hexadiène ou du 2,6-diméthyl-2,6-heptadiène.

3. Procédé selon la revendication 1 ou 2, caractérisé en ce qu'on utilise, comme catalyseurs, des zéolithes du type pentasil.

4. Procédé selon la revendication 3, caractérisé en ce qu'on utilise, comme catalyseurs, des zéolithes d'aluminosilicate du type pentasil.

5. Procédé selon la revendication 3, caractérisé en ce qu'on utilise, comme catalyseurs, des zéolithes de borosilicate du type pentasil.

6. Procédé selon la revendication 3, caractérisé en ce qu'on utilise, comme catalyseurs, des zéolithes de ferrosilicate du type pentasil.

7. Procédé selon la revendication 3, caractérisé en ce qu'on utilise, comme catalyseurs, des zéolithes d'aluminosilicate des types faujasite, érionite, chabazite et offretite.

8. Procédé selon l'une quelconque des revendications 1 à 7, caractérisé en ce qu'on utilise des catalyseurs zéolithiques qui sont dopés avec des métaux de transition et/ou des métaux précieux et/ou des terres rares et/ou des métaux alcalins et/ou des métaux alcalino-terreux.
